# EUROPEAN PATENT APPLICATION

(11) **EP 1 717 308 A1**
(43) Date of publication of application: **02.11.2006**
(21) Application number: 04748265.8
(22) Date of filing: 30.07.2004
(51) Int. Cl.: C12M 1/38

(54) **TEMPERATURE CONTROL DEVICE**

(30) Priority: 14.01.2004 JP 2004007011
(71) Applicant: DAIKIN INDUSTRIES, LTD., Osaka-shi, Osaka 530-8323 (JP)
(72) Inventor: MIYAHARA, Seiichiro, Daikin Industries, Ltd., Tukuba-shi, Ibaraki 305-0481 (JP)
(74) Representative: Goddar, Heinz J.
(86) International application number: PCT/JP2004/011294
(87) International publication number: WO 2005/068608

(57) **Abstract**

The present invention has an object to selectively culture either one of mold and yeast, both of which are fungi, with priority. In order to attaint the object, a cell parts group 101 is heated by a heating mechanism 102 and cooled by a cooling mechanism 103. These operations are controlled by a heating-and-cooling control unit 104, and for instance, approximately 27°C and 30 to 32°C are switched to be adopted to a culturing temperature. Such temperature is set by a temperature-setting unit 105. By setting the culturing temperature to approximately 27°C and 30 to 32°C, respectively, fungi and yeast will easily be selectively cultured with priority, respectively, in culture media provided in the cell parts group 101.

## Description

### Technical Field

The present invention relates to techniques of culturing microorganisms or cells, and more specifically to culturing temperatures therefor.

### Background Art

For microorganism culture, various kinds of culturing methods such as culturing temperatures have been proposed for each microorganism. For instance, the following document 1 gives by way of example 20 to 25°C for fungi (mold and yeast), 35 to 37°C for general bacteria, and 44.5°C for fecal coliform group.

In addition, culturing temperatures for strains can be obtained on a site indicated as the following document 2, for example.

Document 1: The Ministry of Health and Welfare, Environmental Health Bureau, "Guidelines on Food Hygiene Examination, Microorganisms Part", Japan Food Hygiene Association (Corp.), pages 31, 79, 88, 259

Document 2: "IFO biological resources database search", [online], Institute for Fermentation, Osaka, [searched December 3, 2003], Internet <URL: http://www.ifo.or.jp/ifodb/wz02.db_j01>;

### Disclosure of Invention

Technique has been nonexistent, however, of culturing specific microorganisms or cells, particularly mold and yeast, both of which are fungi, at suitable culturing temperatures, respectively. The result has been that neither one of them is selectively cultured with priority.

The present invention has been made in view of the above respects, and has an object to selectively culture specific microorganisms or cells, particularly either one of mold and yeast, both of which are fungi, with priority.

In a first aspect of the present invention, a temperature control device (100, 100A, 100B, 100C) cultures microorganisms or cells at a predetermined culturing temperature. The temperature control device is capable of adopting at least approximately 27°C and 30 to 32°C as the predetermined culturing temperature by switching between those temperatures.

According to the temperature control device of the first aspect, an appropriate culturing temperature can be adopted depending on the type of microorganisms or cells, so that a specific kind of microorganism or cell, particularly either one of mold and yeast in fungi, can easily be selectively cultured with priority.

In a second aspect of the present invention, a temperature control device (100, 100A, 100B, 100C) is the temperature control device according to the first aspect. The temperature control device includes a plurality of temperature control devices that can be connected to each other, and includes a communication unit (107) controlled by a control device (200).

In a third aspect of the present invention, a temperature control device (100, 100A, 100B, 100C) is the temperature control device according to the first aspect. A plurality of the temperature control devices can be connected to each other with the predetermined culturing temperatures being set independently, a specific one (100A) of the plurality is controlled by a control device (200) when the plurality are connected to each other, and the temperature control devices (100B, 100C) other than the specific one are controlled by the specific one.

In a fourth aspect of the present invention, a temperature control device (100, 100A, 100B, 100C) is the temperature control device according to the third aspect. The specific one (100A) manages addresses of the temperature control devices (100B, 100C) other than the specific one, the addresses being viewed from the control device.

In a fifth aspect of the present invention, a temperature control device (100, 100A, 100B, 100C) is the temperature control device according to the first aspect. A plurality of the temperature control devices can be connected to each other with the predetermined culturing temperatures being set independently, data obtained in a specific one (100A) of the plurality is sent to a control device (200) when the plurality are connected to each other, and the temperature control devices (100B, 100C) other than the specific one send their respective data to the specific one.

According to the temperature control devices of the second to fifth aspects, microorganisms or cells under different culturing temperatures can be cultured in parallel by using a single control device.

In a sixth aspect of the present invention, a temperature control device (100, 100A, 100B, 100C) is the temperature control device according to the first aspect. A plurality of the temperature control devices can be connected to each other with the predetermined culturing temperatures being set independently, and each of the plurality is controlled independently by a control device (200) when the plurality are connected to each other.

In a seventh aspect of the present invention, a temperature control device (100, 100A, 100B, 100C) is the temperature control device according to the first aspect. A plurality of the temperature control devices can be connected to each other with the predetermined culturing temperatures being set independently, and data obtained in each of the plurality is sent independently to a control device (200) when the plurality are connected to each other.

According to the temperature control devices of the sixth and seventh aspects, data obtained in the respective temperature control devices can be managed by a single control device.

In an eighth aspect of the present invention, a temperature control device (100, 100A, 100B, 100C) cultures microorganisms or cells at a predetermined culturing temperature. The temperature control device is capable of adopting approximately 27°C as the predetermined culturing temperature.

According to the temperature control device of the eighth aspect, a pace of culturing mold can be increased, contributing to rapid, and specifically, mold can easily be selectively cultured with priority to yeast among fungi.

In a ninth aspect of the present invention, a temperature control device (100, 100A, 100B, 100C) cultures microorganisms or cells at a predetermined culturing temperature. The temperature control device is capable of adopting 30 to 32°C as the predetermined culturing temperature.

According to the temperature control device of the ninth aspect, a pace of culturing yeast can be increased, contributing to rapid culture, and specifically, yeast can easily be selectively cultured with priority to mold among fungi.

In a tenth aspect of the present invention, a temperature control device (100, 100A, 100B, 100C) is the temperature control device according to any one of the first to ninth aspects, and cultures microorganisms or cells at a predetermined culturing temperature. The temperature control device is further capable of adopting 42 to 44.5°C as the predetermined culturing temperature.

According to the temperature control device of the tenth aspect, a pace of culturing colon bacilli can be increased, contributing to rapid culture.

In an eleventh aspect of the present invention, a temperature control device (100, 100A, 100B, 100C) is the temperature control device according to any one of the first to tenth aspects, and cultures microorganisms or cells at a predetermined culturing temperature. The temperature control device is further capable of adopting 35 to 37°C as the predetermined culturing temperature.

According to the temperature control device of the eleventh aspect, a pace of culturing general bacteria can be increased, contributing to rapid culture.

These and other objects, features, aspects and advantages of the present invention will become more apparent from the following detailed description of the present invention when taken in conjunction with the accompanying drawings.

### Brief Description of Drawings

Figs. 1 and 2 show graphs indicating the relationship between the number of bacteria of mold and its detection time.
Figs. 3 and 4 show graphs indicating the relationship between elapsed time of culture and the number of bacteria of yeast.
Fig. 5 is a block diagram showing the structure of a temperature control device.
Fig. 6 is a schematic view showing a state where a plurality of temperature control devices are connected to each other.
Fig. 7 is a schematic view showing a state where a plurality of temperature control devices are connected to each other.

### Best Mode for Carrying Out the Invention

### A. Setting of Appropriate Culturing Temperature

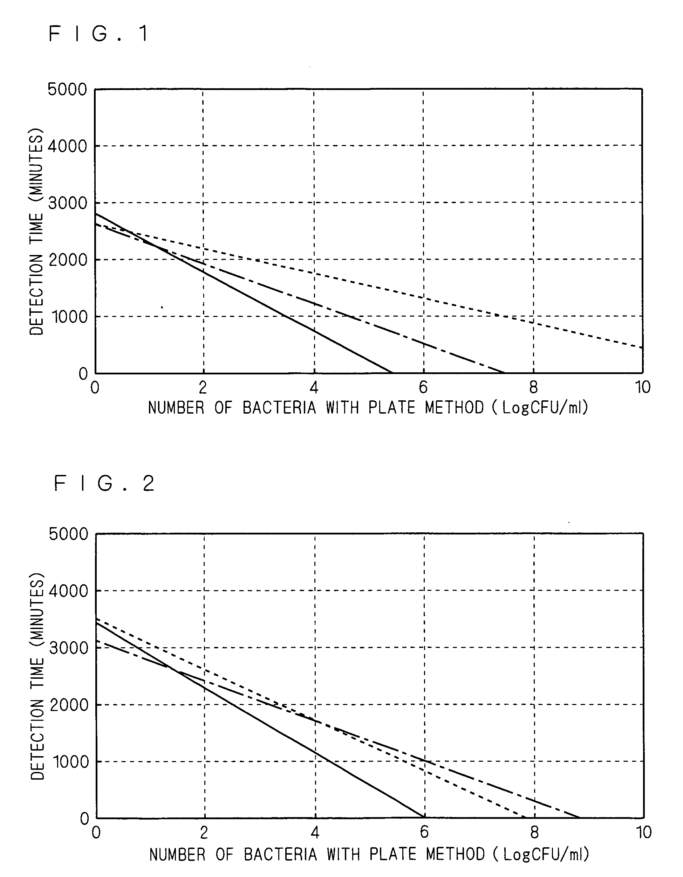

Figs. 1 and 2 show graphs indicating the relationship between the number of bacteria (LogCFU / ml) of mold and its detection time (minutes) using a plate method. In either of the drawings, the dashed line, solid line, and alternate long and short dash line indicate the cases where a culturing temperature is 25°C, 27°C, and 30°C, respectively. Figs. 1 and 2 show the cases where *Aspergillus niger* (6341) and *Penicillium funiculosum* (6345) are adopted as a strain of mold, respectively (the numbers in parentheses indicative of strains represent the IFO numbers adopted by the Institute for Fermentation, Osaka: ditto below).

As understood from the illustration of Figs. 1 and 2, a pace of culture of the mold reaches a maximum at a temperature of 27°C. It is therefore desirable that approximately 27°C be adopted as a culturing temperature for the mold instead of the culturing temperature of 20 to 25°C for fungi culture mentioned in the non-patent document 1, or the culturing temperature of 24°C for the above mold mentioned in the non-patent document 2.

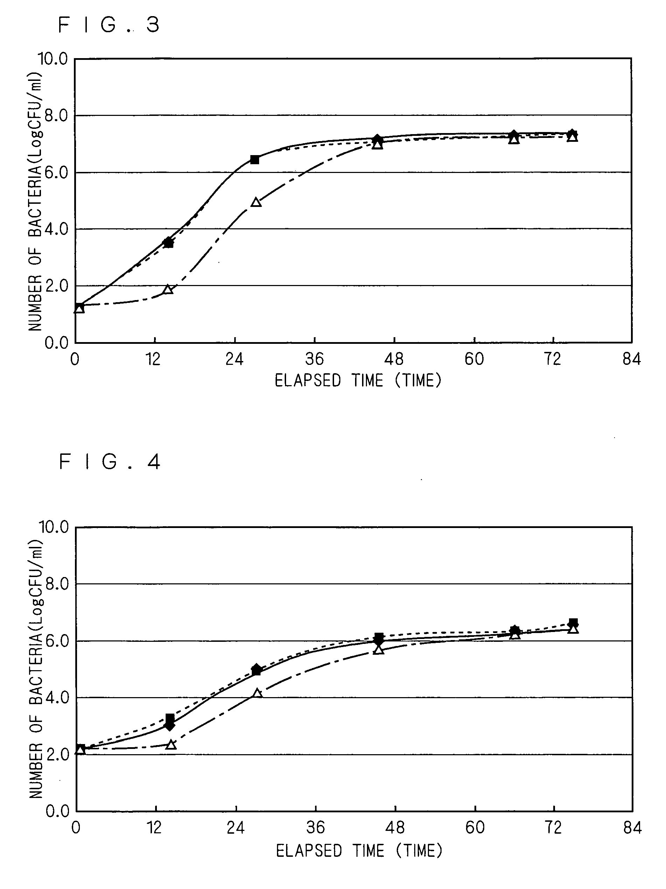

Figs. 3 and 4 show graphs indicating the relationship between elapsed time (time) of culture and the number of bacteria (LogCFU / ml) of yeast. In either of the drawings, the alternate long and short dash line, dashed line, and solid line indicate the cases where a culturing temperature is 25°C, 30°C, and 32°C, respectively. Figs. 3 and 4 show the cases where *Candida albicans* (1594) and *Saccharomyces cerevisiae* (10217) are adopted as a strain of yeast, respectively.

As understood from the illustration of Figs. 3 and 4, a pace of culture of the yeast is higher at a culturing temperature of 30 to 32°C than at a culturing temperature of 25°C. It is therefore desirable that 30°C or more be adopted as a culturing temperature for the yeast instead of the culturing temperature of 20 to 25°C for fungi culture mentioned in the non-patent document 1, or the culturing temperature of 24°C for the above yeast mentioned in the non-patent document 2.

Meanwhile, 35°C or more has been adopted as a culturing temperature for general bacteria, as mentioned above, which is not a desirable temperature from the viewpoint of selectively culturing yeast. After all, it is desirable that a culturing temperature for the yeast be 30 to 32°C.

In view of the foregoing, either one of the mold and yeast can be selected and cultured with priority by adopting approximately 27°C and 30 to 32°C as a culturing temperature.

### B. Structure of Temperature Control Device

Considering the results obtained in "A. Setting of Appropriate Culturing Temperature" described above, it is desirable to perform temperature control capable of adopting at least approximately 27°C and 30 to 32°C as a culturing temperature by switching between those temperatures. An appropriate culturing temperature depending on the type of microorganisms or cells, particularly either one of mold and yeast in fungi can easily be selectively cultured with priority.

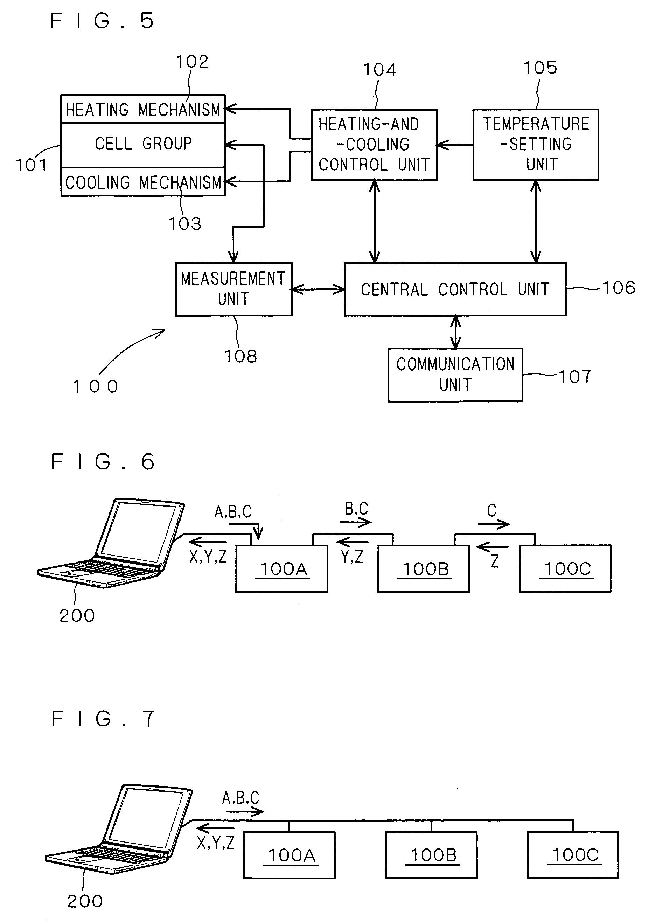

Fig. 5 is a block diagram showing the structure of a temperature control device 100 performing such temperature control. The temperature control device 100 includes a cell parts group 101, a heating mechanism 102, a cooling mechanism 103, a heating-and-cooling control unit 104, and a temperature-setting unit 105.

The cell parts group 101 includes a single or a plurality of cell parts, each of which holds a culture medium for culturing a microorganism or a cell. The cell parts group 101 is heated and cooled by the heating mechanism 102 and the cooling mechanism 103, respectively, to be set to a desirable temperature. With this temperature setting, the temperature control described above can be performed on the culture media provided in the cell parts group 101.

A linear heater or a sheet heater may be adopted by way of example as the heating mechanism 102. Further, a heat block may be used to improve thermal capacity and thus constant-temperature capability. A fan or a Peltier element may be adopted by way of example as the cooling mechanism 103.

The heating-and-cooling control unit 104 controls the operations of the heating mechanism 102 and the cooling mechanism 103 based on a temperature set by the temperature-setting unit 105. The temperature-setting unit 105 is capable of setting at least either one of approximately 27°C and 30 to 32°C. It is further desirable that the culturing temperature be settable to 35 to 37°C that is suitable for culturing general bacteria, and 42 to 44.5°C that is suitable for examining colon bacilli. It is still further desirable that the culturing temperature be settable in a continuous temperature range that includes at least approximately 27°C and 30 to 32°C, and preferably includes 35 to 37°C and 42 to 44.5°C.

It is additionally desirable to provide a central control unit 106 collectively controlling the operations of the heating-and-cooling control unit 104 and the temperature-setting unit 105. This can be organized by adopting conventional techniques. For instance, a microcomputer can be adopted as the central control unit 106.

It is desirable that the temperature control device 100 further include a measurement unit 108 measuring a state of culture in the cell parts group 101. This can be operated under the control of the central control unit 106.

### C. Connection of a Plurality of Temperature Control Devices

It is desirable that the temperature control device 100 further include a communication unit 107. A plurality of temperature control devices 100 are thus connected to each other while setting culturing temperatures independently.

Fig. 6 is a schematic view showing a state where a plurality of temperature control devices 100A, 100B and 100C are connected to each other. The temperature control device 100 described above can be adopted as each of the temperature control devices 100A, 100B and 100C.

A specific one of the plurality of temperature control devices 100A, 100B and 100C, the temperature control device 100A, for example, is connected to and controlled by a single control device 200. For instance, the communication unit 107 of the temperature control device 100A is controlled by the control device 200. More specifically, the control device 200 supplies the communication unit 107 with an instruction A for a culturing temperature to be set by the temperature-setting unit 105 of the temperature control device 100A, an instruction B for a culturing temperature to be set by the temperature-setting unit 105 of the temperature control device 100B, and an instruction C for a culturing temperature to be set by the temperature-setting unit 105 of the temperature control device 100C.

In the temperature control device 100A, the central control unit 106 selects the instruction A intended for the temperature control device 100A from among the instructions A, B and C supplied to the communication unit 107, and supplies the selected instruction to the temperature-setting unit 105.

At least the instructions B and C of the instructions A, B and C supplied to the communication unit 107 of the temperature control device 100A are supplied to the communication unit 107 of the temperature control device 100B.

In the temperature control device 100B, the central control unit 106 selects the instruction B intended for the temperature control device 100B between the instructions B and C (or the instructions A, B and C) supplied to the communication unit 107, and supplies the selected instruction to the temperature-setting unit 105.

At least the instruction C between the instructions B and C (or the instructions A, B and C) supplied to the communication unit 107 of the temperature control device 100B is supplied to the communication unit 107 of the temperature control device 100C.

In the temperature control device 100C, the central control unit 106 selects the instruction C intended for the temperature control device 100C from the instruction C (or the instructions B and C, or the instructions A and C, or the instructions A, B and C) supplied to the communication unit 107, and supplies the selected instruction to the temperature-setting unit 105.

In this manner, microorganisms or cells under different culturing temperatures can be cultured in parallel by using the single control device 200.

Moreover, data indicative of the state of culture measured by the measurement unit 108 in each of the temperature control devices 100A, 100B and 100C can be managed by using the single control device 200. In the temperature control device 100C, for example, the central control unit 106 causes the communication unit 107 to output data Z measured by the measurement unit 108.

In the temperature control device 100B, the communication unit 107 receives the data Z. In addition, the central control unit 106 causes the communication unit 107 to output data Y measured by the measurement unit 108 together with the data Z.

In the temperature control device 100A, the communication unit 107 receives the data Y and Z. In addition, the central control unit 106 causes the communication unit 107 to output data X measured by the measurement unit 108 together with the data Y and Z.

Fig. 7 is a schematic view showing another state where a plurality of temperature control devices 100A, 100B and 100C are connected to each other. Each of the temperature control devices 100A, 100B and 100C can be controlled independently by the control device 200 via the control of the communication unit 107, and can also send independently the data obtained therein to the control device 200.

Further, the temperature control device 100A may manage addresses, which are viewed from the control device 200, of the temperature control devices 100B and 100C other than the temperature control device 100A. In this case, the temperature control device 100A serves as the so-called master machine, and the temperature control devices 100B and 100C serve as the so-called slave machines, respectively.

For mutual transmission and reception among the control device 200 and the temperature control devices 100A, 100B and 100C, a common general protocol (SCSI) may be adopted, or a special protocol may alternatively be used. In such case, the communication units 107 have functions that comply with the protocol.

When the temperature control device 100A serves as a master machine and the temperature control devices 100B and 100C serve as slave machines, respectively, two kinds of protocols may be used together. For instance, RC-232C may be adopted as a protocol to be used for transmission and reception between the control device 200 and the temperature control device 100A, while RS-485 may be adopted as a protocol to be used for transmission and reception between the temperature control device 100A and the temperature control device 100B (or 100C), respectively.

The protocols mentioned above may of course be in either one of serial and parallel modes.

In this manner, data about the culture of microorganisms or cells under different culturing temperatures can be managed by using the single control device 200.

In collecting the data, the setting of a culturing temperature in each of the temperature control devices 100A, 100B, and 100C may be made manually.

By setting the culturing temperatures to 35°C, 42°C and 27°C as the above instructions A, B and C, respectively, the temperature control devices 100A, 100B and 100C can appropriately perform a general bacteria examination, a colon bacilli examination, and a mold examination, respectively.

Furthermore, by setting the culturing temperatures to 35°C, 30°C and 27°C as the above instructions A, B, and C, respectively, the temperature control devices 100A, 100B and 100C can appropriately perform a general bacteria examination, an yeast examination, and a mold examination, respectively.

While the invention has been shown and described in detail, the foregoing description is in all aspects illustrative and not restrictive. It is therefore understood that numerous modifications and variations can be devised without departing from the scope of the invention.

## Claims

1. A temperature control device (100, 100A, 100B, 100C) culturing microorganisms or cells at a predetermined culturing temperature,
said temperature control device being capable of adopting at least approximately 27°C and 30 to 32°C as said predetermined culturing temperature by switching between those temperatures.

2. The temperature control device (100, 100A, 100B, 100C) according to claim 1, wherein
said temperature control device includes a plurality of temperature control devices that can be connected to each other,
said temperature control device comprising a communication unit (107) controlled by a control device (200).

3. The temperature control device according to claim 1, wherein
a plurality of said temperature control devices can be connected to each other with said predetermined culturing temperatures being set independently,
a specific one (100A) of said plurality is controlled by a control device (200) when said plurality are connected to each other, and
said temperature control devices (100B, 100C) other than said specific one are controlled by said specific one.

4. The temperature control device according to claim 3, wherein
said specific one (100A) manages addresses of said temperature control devices (100B, 100C) other than said specific one, said addresses being viewed from said control device.

5. The temperature control device according to claim 1, wherein
a plurality of said temperature control devices can be connected to each other with said predetermined culturing temperatures being set independently,
data obtained in a specific one (100A) of said plurality is sent to a control device (200) when said plurality are connected to each other, and
said temperature control devices (100B, 100C) other than said specific one send their respective data to said specific one.

6. The temperature control device according to claim 1, wherein
a plurality of said temperature control devices can be connected to each other with said predetermined culturing temperatures being set independently, and
each of said plurality is controlled independently by a control device (200) when said plurality are connected to each other.

7. The temperature control device according to claim 1, wherein
a plurality of said temperature control devices can be connected to each other with said predetermined culturing temperatures being set independently, and
data obtained in each of said plurality is sent independently to a control device (200) when said plurality are connected to each other.

8. A temperature control device (100, 100A, 100B, 100C) culturing microorganisms or cells at a predetermined culturing temperature,
said temperature control device being capable of adopting approximately 27°C as said predetermined culturing temperature.

9. A temperature control device (100, 100A, 100B, 100C) culturing microorganisms or cells at a predetermined culturing temperature,
said temperature control device being capable of adopting 30 to 32°C as said predetermined culturing temperature.

10. The temperature control device (100, 100A, 100B, 100C) according to any one of claims 1 to 9,
said temperature control device being further capable of adopting 42 to 44.5°C as said culturing temperature.

11. The temperature control device (100, 100A, 100B, 100C) according to any one of claims 1 to 9,
said temperature control device being further capable of adopting 35 to 37°C as said culturing temperature.

12. The temperature control device (100, 100A, 100B, 100C) according to claim 10,
said temperature control device being further capable of adopting 35 to 37°C as said culturing temperature.
